# EUROPEAN PATENT SPECIFICATION

(11) **EP 3 174 544 B1**
(45) Date of publication and mention of the grant of the patent: **09.12.2020**
(21) Application number: 15759942.4
(22) Date of filing: 27.07.2015
(51) Int. Cl.: A61K 31/685, A61P 25/00, A61P 25/28, A61P 25/18, A61P 25/24

(54) **ESTER OF A PHOSPHOLIPID WITH CONJUGATED LINOLEIC ACID FOR THE TREATMENT OF PSYCHIATRIC DISORDERS WITH NEUROINFLAMMATORY AND NEURODEGENERATIVE BASIS**
ESTER EINES PHOSPHOLIPIDS MIT KONJUGIERTER LINOLSÄURE ZUR BEHANDLUNG VON PSYCHIATRISCHEN STÖRUNGEN MIT NEUROINFLAMMATORISCHER UND NEURODEGENERATIVER BASIS
ESTER D'UN PHOSPHOLIPIDE AVEC UN ACIDE LINOLÉIQUE CONJUGUÉ POUR LE TRAITEMENT DE TROUBLES PSYCHIATRIQUES PRÉSENTANT UNE BASE NEUROINFLAMMATOIRE ET NEURODÉGÉNÉRATIVE

(30) Priority: 31.07.2014 IT RM20140433
(43) Date of publication of application: 07.06.2017
(73) Proprietor: UNIVERSITA' DEGLI STUDI DI CAGLIARI, 09124 Cagliari (IT)
(72) Inventor: BANNI, Sebastiano, I-09124 Cagliari (IT); MELIS, Miriam, I-09124 Cagliari (IT); PISTIS, Marco, I-09124 Cagliari (IT); SOGOS, Valeria, I-09124 Cagliari (IT)
(74) Representative: Primiceri, Maria Vittoria
(86) International application number: PCT/IB2015/055664
(87) International publication number: WO 2016/016790

(56) References cited:
- WO-A1-03/068210
- WO-A2-01/39744
- WO-A2-2004/048504
- WO-A2-2007/068434
- KIMBERLY J. JENKO ET AL: "Conjugated Linoleic Acids and CLA-Containing Phospholipids Inhibit NO Formation in Aortic Endothelial Cells", LIPIDS, vol. 43, no. 4, 12 March 2008 (2008-03-12) , pages 335-342, XP055178108, ISSN: 0024-4201, DOI: 10.1007/s11745-008-3160-y
- DATABASE WPI Week 201322 Thomson Scientific, London, GB; AN 2012-L19837 XP002737578, & PL 398 022 A1 (UNIV WROCLAWIU PRZYRODNICZY) 2 July 2012 (2012-07-02)
- DATABASE WPI Week 201315 Thomson Scientific, London, GB; AN 2012-L19843 XP002737579, & PL 398 021 A1 (UNIV WROCLAWIU PRZYRODNICZY) 2 July 2012 (2012-07-02)
- DATABASE CA [Online] CHEMICAL ABSTRACTS SERVICE, COLUMBUS, OHIO, US; NIEZGODA, NATALIA ET AL: "Synthesis of phosphatidylcholine with conjugated linoleic acid and studies on its cytotoxic activity", XP002737580, retrieved from STN Database accession no. 2013:459598 & NIEZGODA, NATALIA ET AL: "Synthesis of phosphatidylcholine with conjugated linoleic acid and studies on its cytotoxic activity", AUSTRALIAN JOURNAL OF CHEMISTRY , 66(3), 354-361 CODEN: AJCHAS; ISSN: 0004-9425, 2013, DOI: 10.1071/CH12404 10.1071/CH12404

## Description

The present invention relates to an ester of a phospholipid with conjugated linoleic acid for the treatment of psychiatric disorders with neuroinflammatory and neurodegenerative basis.

More in detail, the invention relates to an ester of a phospholipid with conjugated linoleic acid for use in therapeutic treatment of or as a food supplement for psychiatric disorders with neuroinflammatory and neurodegenerative basis, such as depression and schizophrenia.

Depression is bound to give the second largest contribution to the global load of diseases and disabilities within 2020 (World Health Organization), although it still represents a significant unmet clinical need. Studies that have employed modern techniques for evaluating depressive episodes in general population agree on the fact that 13-16% of adults have at least once in their life met the criteria for the Major Depressive Disorder (Kessler et al., 2003; Hasin et al., 2005), perhaps even reaching 20% when this datum is extrapolated by relating it to the whole length of life. In these samples of general population, the average depressive episode lasts 3 to 4 months (Spijker et al., 2002; Kessler et al., 2003; Eaton et al., 2008). About half the people who have suffered from it once then recovers and have no relapse (Eaton et al., 2008), even though approx. 20% of the depressive episodes have a chronic course lasting two years or longer (Spijker et al., 2002). These data reveal that depression cases in general population, about half of which are not diagnosed or treated (Kessler et al., 2003; Hasin et al., 2005; Eaton et al., 2008), are more common, have a shorter duration, and are often less recurring than emerges from studies conducted on clinical samples.

Schizophrenia (SZ) is a progressive neuropsychiatric disease that affects, in average, 1% of the population, and causes diffused compromission of behaviours and cognitive functions (Pantelis and Brewer, 1995). Cognitive deficits are present independently of the stage of development of the disease, and are particularly serious as concerns the tasks related to the operation of the frontal and temporal lobes, including attention, speed, executive functions, verbal memory and learning (Censits et al., 1997; Townsend et al., 2001). A certain number of neurobiological alterations have been associated with cognitive compromission, such as loss of white and gray matter and frontal lobe hypofunction (Weinberger et al., 1988; Price et al., 2010; de Castro-Manglano et al., 2011). Various molecular modifications have also been associated with the disease, including loss of synaptic proteins of the brain and increased neuroinflammation, excitotoxicity and arachidonic-acid metabolism markers (AA) (Chen et al., 2011; Rao et al., 2011b; Rao et al., 2011a; Rao et al., 2012). Similar modifications of these markers have also been found in dementia associated with HIV-1 (Everall et al., 1999; Aoki et al., 2005) and in bipolar disorder (Aoki et al., 2005; Kim et al., 2010).

In light of the above, it is apparent that there is a need for new methods of treatment of psychiatric disorders with neuroinflammatory and neurodegenerative basis, which can overcome the drawbacks of the therapies known in the art.

Some evidence suggests that depression is often triggered by stressful life events, that stress triggers neuronal microtraumatisms and neuroinflammatory activation in the brain, and that inflammatory mediators can induce depressive symptoms (Wager-Smith and Markou, 2011). Furthermore, it has recently being emerging that anti-inflammatory treatments have antidepressant effects, and that antidepressant therapies can improve, in addition to neurogenesis, also the effects of neurotrophines and neuronal plasticity (Wager-Smith and Markou, 2011).

Inflammation is an active defense reaction against different insults, which aims at neutralizing noxious agents. Although inflammation is useful as a protective function for controlling infections and promoting tissue repair, it may also cause damage to tissues (Layé, 2010). This mediated response of the brain leads, in particular, to profound metabolic alterations, in the form of a higher thermoregulatory set-point resulting in fever, and drastic behavioural changes commonly labelled as "diseased behaviour" (anorexy, locomotor activity reduction, social withdrawal, etc.). The expression of cytokines in the brain plays a key role in the physiopathology of immune neurological disorders (e.g. multiple sclerosis) and non-immune ones (e.g. brain damage, ictus, Alzheimer disease) (Layé, 2010).

In the brain, inflammatory mediators are mainly produced by endothelial and glial cells, including astrocytes and microglia (Rothwell and Luheshi, 2000). The expression of pro-inflammatory cytokines in the brain increases in response to various conditions, such as infections (bacteria, viruses), lesions, traumas and oxidative stress. Neuroinflammation, i.e. the inflammatory response in the brain, has many cellular and biochemical characteristics that make it different from peripheral inflammatory response.

Among the functional consequences of neuroinflammation we include cognitive and behavioural alterations, which usually occur in the absence of neurotoxicity (Dantzer, 2001). It is well known that the behavioural repertoire of human beings and animals changes drastically in the course of an infection. Diseased individuals are scarcely motivated to eat, are lazy, complain of tiredness and discomfort, lose interest in social activities, and exhibit significant sleep pattern changes. In addition, they feel sick and, being afflicted by pain, show inability to experience pleasure (anhedonia) and exhibit attention, concentration and memory difficulties (Dantzer et al., 2008). These alterations are responsible for a reduced quality of life and welfare, and can be reproduced in healthy individuals by means of peripheral or central injections of pro-inflammatory cytokines (Kent et al., 1996). When neuroinflammation is exacerbated or prolonged, it may lead to neuronal cellular death and neurodegeneration as a consequence of deprivation of neuronal growth factors or overproduction of reactive species of oxygen (Rothwell and Luheshi, 2000; Venters et al., 2000).

Neuroinflammation has many aspects, all of which occur simultaneously. Following exposition to harmful stimuli, some neuroinflammatory components include microglial activation, cytokine release, and induction of tissue repair enzymes, which together limit the cellular damage and promote the repair. At behavioural level, cytokine-induced diseased behaviour is nothing else than the exterior manifestation of a central motivational state that helps the organism fight the infection and promote the recovery (Dantzer, 2001). Pro-inflammatory cytokines act in the brain to induce non-specific infection symptoms, including fever and profound psychological and behavioural changes defined as "disease behaviour". Affected subjects exhibit weakness, discomfort, cognitive alterations and laziness, hypersleep, depressed activity, and loss of interest in social activities (Dantzer, 2001). Although these symptoms are generally considered to be the result of the debilitating process occurring during the infection, they are actually part of a natural homeostatic reaction of the body for fighting against infections (Hart, 1988). These behavioural changes have been proved to be the expression of a motivational state that resets the priorities of the body to promote resistance to pathogens and recovery from the infection. Since it prevents metabolically costly activities and favours the expression of activities that promote calorie preservation (e.g. rest), diseased behaviour positively contributes to inflammation recovery (Dantzer, 2001).

There is much evidence in favour of a role played by cytokines in the mediation of mood disorders and cognitive disorders developing in patients treated with cytokines by immunotherapy (Capuron and Dantzer, 2003). The same mechanisms seem to operate for the wide variety of non-specific disease symptoms developing in patients suffering from somatic diseases characterized by an inflammatory component, including coronary disease, rheumatoid arthritis, asthma, cancer, ictus, and various neuropathologies (Eikelenboom et al., 2002; Gidron et al., 2002; Kiecolt-Glaser and Glaser, 2002; Cleeland et al., 2003). Many patients complain for pain, tiredness, anorexy, sleep disorders, and cognitive and mood disorders. These non-specific neurovegetative and psychiatric symptoms are not necessarily the result of a chain of events connected to each other by direct causality (e.g. pain causes sleep disorders, which have a negative impact on cognition and induce tiredness and exhaustion, leading to anorexy) (Cleeland et al., 2003). They may actually represent only another aspect of the inflammatory process. These non-specific symptoms are the main source of suffering for the patient, often more than the diseased organ.

Several studies have reported microglial activation (Steiner et al., 2008; van Berckel et al., 2008) and high levels of pro-inflammatory cytokines in post-mortem brain tissue of SZ patients, as well as high levels of cytokines in the plasma (Licinio et al., 1993; Drexhage et al., 2008; Muller and Schwarz, 2008). Microglial and astrocyte activation may induce, via the nuclear factor kappa B (NF-kB) pathway, release of pro-inflammatory cytokines (interleukine-1 beta (IL-1β)) and of the tumor necrosis factor alpha (TNFα), and also activation of numerous signal transduction pathways, including the AA cascade (Hernandez et al., 1999; Laflamme et al., 1999; Blais and Rivest, 2001; Moolwaney and Igwe, 2005). Chronic activation of the N-methyl-D-aspartate (NMDA) receptor causes selective over-regulation of mRNA, proteins, and activity of cytosolic phospholipase A2 (cPLA2) selective for AA in rat brain (Rao et al., 2007). During excitotoxic insults, specific excitotoxicity biomarkers, such as inducible nitric oxide synthase (iNOS) (Acarin et al., 2002) and c-Fos, are expressed in the brain (Rogers et al., 2005).

AA is mostly located in the position (sn)-2 of membrane phospholipids, from where it can be hydrolized by cPLA2 or by secretory sPLA2. The released AA can be converted, under the action of cyclooxygenase (COX), lipooxygenase (LOX) and thromboxane synthase (TXS) enzymes, into pro-inflammatory mediators, such as prostaglandins (PG) H2 and leukotrienes. The increase in AA brain markers has been reported in an experimental model of neuroinflammation in rats (Rosenberger et al., 2004; Basselin et al., 2011).

An association has been demonstrated, both *in vivo* and *in vitro,* between AA and its pro-inflammatory metabolytes and neuronal apoptosis and synapse loss (Okuda et al., 1994; Williams et al., 1998; Farooqui et al., 2001; Yagami et al., 2002; Fang et al., 2008). Also, the reduced density of dendritic spines and their complexity have been associated with deficits in learning, memory, and general cognitive functions (Masliah et al., 1997). In the SZ brain, associations between synapse loss, elevated AA cascade markers, neuroinflammation and loss of synaptic proteins have not yet been clearly identified as a pathogenetic characteristic of the disease. However, previous studies have reported structural, metabolic and neurotransmission anomalies in the frontal cortex of SZ patients (Weinberger et al., 1988; Beasley et al., 2009; Price et al., 2010; de Castro-Manglano et al., 2011).

Sustained neuroinflammation may also be one of the factors that trigger the development of depressive disorder and may be involved in the mediation of cellular apoptosis, which is the classic form of programmed cell death.

It has also been hypothesized that the progression of the disease and the cognitive deficits found in SZ may be associated with neuroinflammation and AA cascade activation; therefore, anti-neuroinflammatory therapies might be therapeutically useful.

An important anti-inflammatory role is played at brain level by peroxisomes via degradation of the pro-inflammatory eicosanoids derived from arachidonic acid by beta-oxidation (Diczfalusy et al., 1991; Diczfalusy et al., 1993; Ferdinandusse et al., 2002). Peroxisomial beta oxidation is regulated by a transcription factor, PPAR alpha, which exerts its action after binding with specific ligands (Rakhshandehroo et al., 2010). In addition, PPAR alpha activation plays an important role in the homeostasis of the dopaminergic system (Melis et al., 2010; Melis et al., 2013a; Melis et al., 2013b). Therefore, PPAR alpha might play a direct role in the modulation of the dopaminergic system, and might be the key factor allowing functional recovery of the dopaminergic system after an inflammatory event. On the contrary, non-activation of PPAR alpha might sustain inflammation and loss of homeostatic control of the dopaminergic system. The physiologic ligands of PPAR alpha are fatty acids which, depending on the length of the chain and on the number of double bonds, show different affinity towards this receptor. Therefore, the availability of different fatty acids regulates the activity of PPAR alpha. Eicosanoids derived from arachidonic acid, such as prostaglandins and leukotrienes, are endogenous ligands of PPAR alpha. A physiological mechanism can thus be assumed to exist, wherein the activation of PPAR alpha limits their concentration by increasing their catabolism. In addition, the PPAR alpha activation increases the biosynthesis of the amides of palmitic acid, i.e. palmitoylethanolamide (PEA), and of oleic acid, i.e. oleoylethanolamide (OEA) (Melis et al., 2013a), which in turn are ligands of PPAR alpha. High levels of PEA and OEA sustain PPAR alpha activation, thus ensuring anti-inflammatory activity and preservation of the homeostasis of the dopaminergic system. Therefore, in stressful and inflammatory-insult conditions, high cerebral levels of OEA and PEA might ensure an adequate anti-inflammatory response and neuronal functional preservation.

Conjugated linoleic acid (CLA) is a set of isomeric forms of linoleic acid, such as, for example, the 9c,11t and 10t,12c isomers. CLA is a fatty acid which is present at low concentrations in our diet, and which is an avid ligand of PPAR alpha (Moya-Camarena et al., 1999). CLA has several nutritional properties at peripheral level (Belury, 2002), but also great potentialities at the level of the central nervous system, since has been proved that it crosses the hematoencephalic barrier in both test animal (Fa et al., 2005) and man (Cappa et al., 2012).

Another very important factor that affects the nutritional activity of alimentary fatty acids is the form in which they are present. It has been demonstrated, in fact, that polyunsaturated fatty acids are more bioavailable in phospholipid form than in triglycerid form in many tissues (Batetta et al., 2009), including the brain (Di Marzo et al., 2010).

WO/2003/068210 discloses pharmaceutical, cosmetic and dietetic compositions and functional foods, constituted by: A) phospholipid mixtures containing N-acyl-phosphatidyl-ethanolamines (NAPEs) and/or B) phospholipid mixtures containing N-acyl-ethanol amines (NAEs) together with phosphatidic acids (PAs) and/or lysophosphatidic acids (LPAs) for the preparation of medicaments having anorexic activity or of medicaments or foodstuffs for the treatment of aging, obesity and excess weight; diabetes; cerebro-degenerative disorders such as Alzheimer's disease, Parkinson's disease and senile dementia; stress, depression; tumours; menopausal syndromes; osteoporosis; prostate hypertrophy; skin aging; panniculopathy (cellulitis) and alopecia.

In Kimberly J. Jenko et al. (2008) are disclosed the effects of five conjugated linoleic acid isomers and of non-conjugated linoleic acid on nitric oxide production.

DATABASE WPI XP002737578 discloses phospholipid derivatives of palmitic acid and conjugated linoleic acid comprising conjugated linoleic acid isomers for use in the treatment of cancer.

DATABASE WPI XP002737579 discloses the use of substituted dyacil-sn-glycero-3-phosphocholine compounds with conjugated linoleic acid residue as prodrugs for the treatment of cancer and artherosclerosis.

In WO/2004/048504 are described esters containing conjugated linoleic acids that are used to stimulate arachidonic acid release and having anti-thrombotic effect.

In Niezgoda et al. (2013) is reported how phospholipids with conjugated linoleic acid, that are potential prodrugs, are synthetized. It was also reported their use on cancer cells.

WO 2007/068434 discloses a monoester of a steroid with either a saturated or an unsaturated C₁-C₂₄ fatty acid wherein the fatty acid could be an isomer of conjugated linoleic acid for use as a medicament and more specifically in the treatment of Alzheimer's disease

The inventors of the present invention have now found that conjugated linoleic acid, in particular the c9,t11 isomer, may be able to improve protection against neuroinflammation, induce a re-balance of the dopaminergic neuronal function, and enhance the synaptogenesis and neuritogenesis, thus turning out to be a very good alimentary support in psychiatric disorders with neuroinflammatory and neurodegenerative basis. This effect is much enhanced if CLA is esterified in phospolipids such as phosphatidylcholine, phosphatidylethanolamine, phosphatidylserine, since it is readily incorporated at the level of the central nervous system, where it performs its anti-inflammatory action through PPAR alpha activation and PEA and OEA increase.

Therefore, the results of the above-mentioned study demonstrate that CLA, and in particular the c9,t11 isomer, can increase the levels of PEA and OEA both *ex vivo* and *in vivo,* and hence sustain PPAR alpha activation. The PEA and OEA increase, since it sustains PPAR alpha activation, is crucial for the physiologic response to neuroinflammatory events, in addition to preserving the homeostasis of the dopaminergic system. In fact, CLA can reduce *in vitro* the levels of TNF alpha in both astrocytes and microglia.

The use of CLA in degenerative diseases which are characteristic of old age has been described in United States patent US20090169533, which shows experimental data concerning presbyacusis alone. More specifically, patent US20090169533 relates to the use of CLA as an individual component and/or mixed with alpha lipoic acid (ALA), Q10 and phenylalanine (PA) for prevention and treatment of neurodegenerative diseases, in particular those which are characteristic of old age, such as presbyacusis. The action mechanism hypothesized in this patent is assumed to be correlated to the antioxidant capability of ALA and Q10, and to the role played by PA as a precursor of L-tyrosine, and hence of dopamine, norepinephrine, epinephrine, of the neurotransmitters in the cochlea. On the other hand, CLA is assumed to act through its capability of reducing the body weight, similarly to caloric restriction, which has been proven to prevent presbyacusis (Someya et al. 2007). Therefore, the study described in patent US20090169533 appears to exclusively address neurodegenerative diseases correlated to old age, and is based on antioxidant capabilities and on the effect of caloric restriction for preventing the above-mentioned diseases.

The CLA activity demonstrated in the present invention is completely different from the one described in patent US20090169533. The present invention, in fact, describes a direct activity of CLA at the level of the central nervous system, which is disconnected from a peripheral activity, such as the one on adiposity, or from any activity correlated to neurodegeneration processes which are characteristic of old age. For this reason, the use of CLA in the form of phospholipidic ester is of fundamental importance to ensure the utmost bioavailability at brain level. Moreover, the CLA isomer according to the present invention is c9,t11, which has no body weight reduction activity (Pariza et al. 2001), while the only CLA isomer capable of reducing body fat is t10,c12 (Pariza et al. 2001).

It is therefore a specific object of the present invention to provide an ester of a phospholipid, preferably phosphatidylcholine, phosphatidylethanolamine, phosphatidylserine, with conjugated linoleic acid or conjugated linoleic acid and docosahexaenoic acid (DHA), for use in the neuropsychiatric medical field. Therefore, the ester according to the present invention may consist of a phospholipid esterified with one or two molecules of conjugated linoleic acid or with one molecule of conjugated linoleic acid and one molecule of DHA.

The conjugated linoleic acid is the c9,t11 isomer; according to the present invention one may use, therefore, either a mixture of isomers having a high concentration of said isomer, e.g. at least 80% of said isomer, or the pure isomer.

Esterification of the phospholipid with DHA as well, in addition to CLA, is advantageous because DHA also plays an important role for the brain function (Rapoport et al. 2011). It has been recently demonstrated that the DHA amide has an action that promotes the synaptogenesis and the neuritogenesis (Kim and Spector, 2013). Furthermore, DHA has also been found to be a high-affinity ligand of PPAR alpha (Diep et al., 2000). Therefore, administration of CLA and DHA simultaneously esterified as phospholipids allows them to be readily incorporated at the level of the central nervous system, where they perform their anti-inflammatory function via PPAR alpha activation and PEA and OEA increase, and via enhancement of neurogenesis by increasing the DHA amide.

It is a further object of the present invention to provide an ester of a phospholipid, such as, for example, phosphatidylcholine, phosphatidylethanolamine, phosphatidylserine, with conjugated linoleic acid or conjugated linoleic acid and docosahexaenoic acid (DHA) for use in treatment and prevention of psychiatric disorders, preferably having neuroinflammatory and neurodegenerative basis, including, without being limited to, psychotic disorders such as schizophrenia, schizophreniform disorder, schizoaffective disorder, or mood disorders such as major depression, dysthymic disorder.

Preferably, the conjugated linoleic acid is the c9,t11 isomer.

The present invention also relates to a pharmaceutical composition comprising or consisting of an ester of a phospholipid with conjugated linoleic acid or conjugated linoleic acid and docosahexaenoic acid as active ingredient, in association with one or more pharmaceutically acceptable excipients and/or adjuvants. As aforementioned, the phospholipid can be selected from, for example, phosphatidylcholine, phosphatidylethanolamine, phosphatidylserine. The conjugated linoleic acid is the c9,t11 isomer.

The pharmaceutical composition of the invention can be used as a therapeutic composition or as a food supplement, and may further comprise antipsychotic and/or antidepressant drugs.

A further object of the present invention is the use of the pharmaceutical composition of the invention for treatment and prevention of psychiatric disorders preferably having a neuroinflammatory and neurodegenerative basis, such as, for example, psychotic disorders such as schizoprenia, schizophreniform disorder, schizoaffective disorder, or mood disorders such as major depression, dysthymic disorder.

The pharmaceutical composition of the invention may further comprise DHA.

A preferred embodiment of the present invention will now be described by way of non limiting example with particular reference to the annexed drawings, wherein:

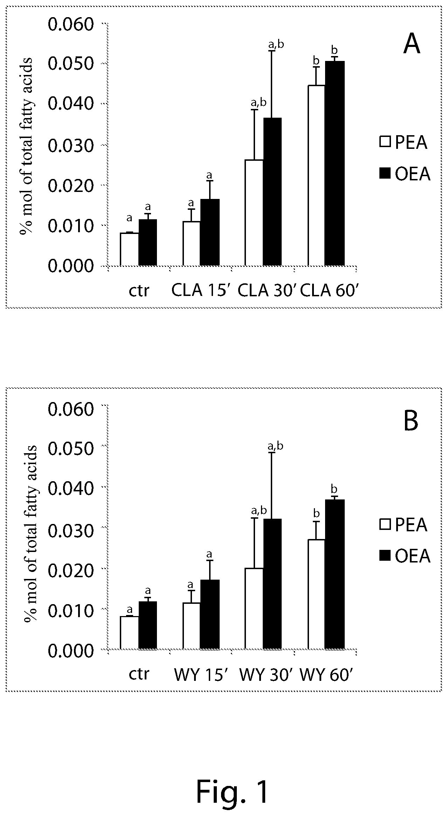

- Figure 1 shows the value of PEA and OEA in rat brain slices incubated with CLA (80% c9,t11 isomer) (A) or WY (B) for 15, 30 or 60 min. The values, mean and DS, are expressed as molar % of the total fatty acids. Different letters indicate significant differences (p<0.05); the One-way ANOVA statistical analysis was used, with Tukey test for post-hoc analysis. Similar results were obtained by using pure (99%) c9,t11 isomer.

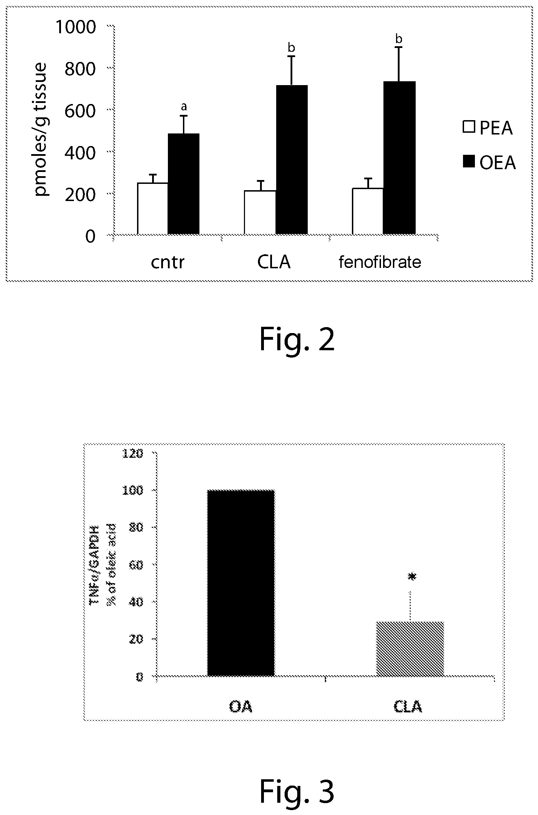

- Figure 2 shows the values of PEA and OEA in the frontal cortex of mice treated for 14 days with a diet containing 0.2% of fenofibrate or administration of CLA (80% c9,t11 isomer) 12 h prior to sacrifice. The values, mean and DS, are expressed as pmoles/g tissue. Different letters indicate significant differences (p<0.05); the One-way ANOVA statistical analysis was used, with Tukey test for post-hoc analysis.
- Figure 3 shows the values of TNF alpha in astrocytes after treatment with oleic acid (OA) or CLA (80% c9,t11 isomer) and subsequent treatment with LPS. The values, mean and DS, are expressed as % of the values obtained with oleic acid. * significantly different (p<0.05) with Student's t-test.

### EXAMPLE 1: Ex vivo and in vivo study on the effect of CLA on the levels of PEA, OEA and TNF alpha

The methods employed for the analysis of the levels of conjugated linoleic acid, PEA and OEA *in vivo* and *in vitro* are described in detail in (Melis et al. 2001) and in (Piscitelli et al. 2011), respectively.

### Results

Studies conducted on brain slices have demonstrated how *ex vivo* incubation with CLA in free form, i.e. both with 80% c9,t11 isomer and with pure isomer, increases the levels of PEA and OEA (Fig. 1A) by about 5 times. On the contrary, WY14643 (WY), a synthetic agonist of PPAR alpha used as a comparison since it is an inductor of PPAR alpha, increases the levels of PEA and OEA by about 3-4 times (Fig. 1B).

In addition, acute *in vivo* administration of CLA (80% c9,t11 isomer), 12 hours prior to sacrifice and collection of brain sections, significantly increases the levels of OEA in the frontal cortex of mice, to a level comparable to that induced by a diet with fenofibrate, another known synthetic agonist of PPAR alpha (Puligheddu et al., 2013) (Fig. 2).

Since astrocytes are involved in neuroinflammation, the action of CLA (80% c9,t11 isomer) was also verified in cultures of astrocytes after having induced neuroinflammation with LPS. Oleic acid was used as a control. The results clearly demonstrate a significant decrease of TNF alpha in cultured astrocytes (Fig. 3).

### Bibliographic references

Acarin L, Peluffo H, Gonzalez B, Castellano B (2002) Expression of inducible nitric oxide synthase and cyclooxygenase-2 after excitotoxic damage to the immature rat brain. Journal of neuroscience research 68:745-754.
Aoki C, Sekino Y, Hanamura K, Fujisawa S, Mahadomrongkul V, Ren Y, Shirao T (2005) Drebrin A is a postsynaptic protein that localizes in vivo to the submembranous surface of dendritic sites forming excitatory synapses. The Journal of comparative neurology 483:383-402.
Basselin M, Ramadan E, Chen M, Rapoport SI (2011) Anti-inflammatory effects of chronic aspirin on brain arachidonic acid metabolites. Neurochemical research 36:139-145. Batetta B, Griinari M, Carta G, Murru E, Ligresti A, Cordeddu L, Giordano E, Sanna F, Bisogno T, Uda S, Collu M, Bruheim I, Di Marzo V, Banni S (2009) Endocannabinoids may mediate the ability of (n-3) fatty acids to reduce ectopic fat and inflammatory mediators in obese Zucker rats. Journal of Nutrition 139:1495-1501.
Beasley CL, Dwork AJ, Rosoklija G, Mann JJ, Mancevski B, Jakovski Z, Davceva N, Tait AR, Straus SK, Honer WG (2009) Metabolic abnormalities in fronto-striatal-thalamic white matter tracts in schizophrenia. Schizophrenia research 109:159-166.
Belury MA (2002) Dietary conjugated linoleic acid in health: Physiological effects and mechanisms of action [Review]. Ann Rev Nutr 22:505-531.
Blais V, Rivest S (2001) Inhibitory action of nitric oxide on circulating tumor necrosis factor-induced NF-kappaB activity and COX-2 transcription in the endothelium of the brain capillaries. Journal of neuropathology and experimental neurology 60:893-905. Cappa M, Bizzarri C, Petroni A, Carta G, Cordeddu L, Valeriani M, Vollono C, De Pasquale L, Blasevich M, Banni S (2012) A mixture of oleic, erucic and conjugated linoleic acids modulates cerebrospinal fluid inflammatory markers and improve somatosensorial evoked potential in X-linked adren.oleukodystrophy female carriers. J Inherit Metab Dis 35:899-907.
Capuron L, Dantzer R (2003) Cytokines and depression: the need for a new paradigm. Brain, behavior, and immunity 17 Suppl 1:S119-124.
Censits DM, Ragland JD, Gur RC, Gur RE (1997) Neuropsychological evidence supporting a neurodevelopmental model of schizophrenia: a longitudinal study. Schizophrenia research 24:289-298.
Chen KH, Reese EA, Kim HW, Rapoport SI, Rao JS (2011) Disturbed neurotransmitter transporter expression in Alzheimer's disease brain. Journal of Alzheimer's disease : JAD 26:755-766.
Cleeland CS, Bennett GJ, Dantzer R, Dougherty PM, Dunn AJ, Meyers CA, Miller AH, Payne R, Reuben JM, Wang XS, Lee BN (2003) Are the symptoms of cancer and cancer treatment due to a shared biologic mechanism? A cytokine-immunologic model of cancer symptoms. Cancer 97:2919-2925.
Dantzer R (2001) Cytokine-induced sickness behavior: mechanisms and implications. Annals of the New York Academy of Sciences 933:222-234.
Dantzer R, O'Connor JC, Freund GG, Johnson RW, Kelley KW (2008) From inflammation to sickness and depression: when the immune system subjugates the brain. Nature reviews Neuroscience 9:46-56.
de Castro-Manglano P, Mechelli A, Soutullo C, Gimenez-Amaya J, Ortuno F, McGuire P (2011) Longitudinal changes in brain structure following the first episode of psychosis. Psychiatry research 191:166-173.
Di Marzo V, Griinari M, Carta G, Murru E, Ligresti A, Cordeddu L, Giordano E, Bisogno T, Collu M, Batetta B, Uda S, Berge K, Banni S (2010) Dietary krill oil increases docosahexaenoic acid and reduces 2-arachidonoylglycerol but not N-acylethanolamine levels in the brain of obese Zucker rats. International Dairy Journal 20:231-235.
Diczfalusy U, Kase BF, Alexson SE, Bjorkhem I (1991) Metabolism of prostaglandin F2 alpha in Zellweger syndrome. Peroxisomal beta-oxidation is a major importance for in vivo degradation of prostaglandins in humans. The Journal of clinical investigation 88:978-984.
Diczfalusy U, Vesterqvist O, Kase BF, Lund E, Alexson SE (1993) Peroxisomal chain-shortening of thromboxane B2: evidence for impaired degradation of thromboxane B2 in Zellweger syndrome. J Lipid Res 34:1107-1113.
Diep QN, Touyz RM, Schiffrin EL (2000) Docosahexaenoic acid, a peroxisome proliferator-activated receptor-alpha ligand, induces apoptosis in vascular smooth muscle cells by stimulation of p38 mitogen-activated protein kinase. Hypertension 36:851-855.
Drexhage RC, Padmos RC, de Wit H, Versnel MA, Hooijkaas H, van der Lely AJ, van Beveren N, deRijk RH, Cohen D (2008) Patients with schizophrenia show raised serum levels of the pro-inflammatory chemokine CCL2: association with the metabolic syndrome in patients? Schizophrenia research 102:352-355.
Eaton WW, Shao H, Nestadt G, Lee HB, Bienvenu OJ, Zandi P (2008) Population-based study of first onset and chronicity in major depressive disorder. Archives of general psychiatry 65:513-520.
Eikelenboom P, Bate C, Van Gool WA, Hoozemans JJ, Rozemuller JM, Veerhuis R, Williams A (2002) Neuroinflammation in Alzheimer's disease and prion disease. Glia 40:232-239.
Esposito G, Giovacchini G, Liow JS, Bhattacharjee AK, Greenstein D, Schapiro M, Hallett M, Herscovitch P, Eckelman WC, Carson RE, Rapoport SI (2008) Imaging neuroinflammation in Alzheimer's disease with radiolabeled arachidonic acid and PET. Journal of nuclear medicine : official publication, Society of Nuclear Medicine 49:1414-1421.
Everall IP, Heaton RK, Marcotte TD, Ellis RJ, McCutchan JA, Atkinson JH, Grant I, Mallory M, Masliah E (1999) Cortical synaptic density is reduced in mild to moderate human immunodeficiency virus neurocognitive disorder. HNRC Group. HIV Neurobehavioral Research Center. Brain pathology 9:209-217.
Fa M, Diana A, Carta G, Cordeddu L, Melis MP, Murru E, Sogos V, Banni S (2005) Incorporation and metabolism of c9,t11 and t10,c12 conjugated linoleic acid (CLA) isomers in rat brain. Biochim Biophys Acta 1736:61-66.
Fang KM, Chang WL, Wang SM, Su MJ, Wu ML (2008) Arachidonic acid induces both Na+ and Ca2+ entry resulting in apoptosis. Journal of neurochemistry 104:1177-1189.
Farooqui AA, Yi Ong W, Lu XR, Halliwell B, Horrocks LA (2001) Neurochemical consequences of kainate-induced toxicity in brain: involvement of arachidonic acid release and prevention of toxicity by phospholipase A(2) inhibitors. Brain research Brain research reviews 38:61-78.
Ferdinandusse S, Meissner T, Wanders RJ, Mayatepek E (2002) Identification of the peroxisomal beta-oxidation enzymes involved in the degradation of leukotrienes. Biochem Biophys Res Commun 293:269-273.
Gidron Y, Gilutz H, Berger R, Huleihel M (2002) Molecular and cellular interface between behavior and acute coronary syndromes. Cardiovascular research 56:15-21.
Harigaya Y, Shoji M, Shirao T, Hirai S (1996) Disappearance of actin-binding protein, drebrin, from hippocampal synapses in Alzheimer's disease. Journal of neuroscience research 43:87-92.
Hart BL (1988) Biological basis of the behavior of sick animals. Neurosci Biobehav Rev 12:123-137.
Hasin DS, Goodwin RD, Stinson FS, Grant BF (2005) Epidemiology of major depressive disorder: results from the National Epidemiologic Survey on Alcoholism and Related Conditions. Archives of general psychiatry 62:1097-1106.
Hatanpaa K, Isaacs KR, Shirao T, Brady DR, Rapoport SI (1999) Loss of proteins regulating synaptic plasticity in normal aging of the human brain and in Alzheimer disease. Journal of neuropathology and experimental neurology 58:637-643.
Hernandez M, Bayon Y, Sanchez Crespo M, Nieto ML (1999) Signaling mechanisms involved in the activation of arachidonic acid metabolism in human astrocytoma cells by tumor necrosis factor-alpha: phosphorylation of cytosolic phospholipase A2 and transactivation of cyclooxygenase-2. Journal of neurochemistry 73:1641-1649.
Huang SK, Klein DC, Korf HW (1992) Immunocytochemical demonstration of rod-opsin, S-antigen, and neuron-specific proteins in the human pineal gland. Cell and tissue research 267:493-498.
Kent S, Bret-Dibat JL, Kelley KW, Dantzer R (1996) Mechanisms of sickness-induced decreases in food-motivated behavior. Neurosci Biobehav Rev 20:171-175.
Kessler RC, Berglund P, Demler O, Jin R, Koretz D, Merikangas KR, Rush AJ, Walters EE, Wang PS, National Comorbidity Survey R (2003) The epidemiology of major depressive disorder: results from the National Comorbidity Survey Replication (NCS-R). JAMA : the journal of the American Medical Association 289:3095-3105.
Kiecolt-Glaser JK, Glaser R (2002) Depression and immune function: central pathways to morbidity and mortality. Journal of psychosomatic research 53:873-876.
Kim HW, Rapoport SI, Rao JS (2010) Altered expression of apoptotic factors and synaptic markers in postmortem brain from bipolar disorder patients. Neurobiology of disease 37:596-603.
Kim HY, Spector AA (2013) Synaptamide, endocannabinoid-like derivative of docosahexaenoic acid with cannabinoid-independent function. Prostaglandins Leukot Essent Fatty Acids 88:121-125.
Kojima N, Kato Y, Shirao T, Obata K (1988) Nucleotide sequences of two embryonic drebrins, developmentally regulated brain proteins, and developmental change in their mRNAs. Brain research 464:207-215.
Laflamme N, Lacroix S, Rivest S (1999) An essential role of interleukin-1beta in mediating NF-kappaB activity and COX-2 transcription in cells of the blood-brain barrier in response to a systemic and localized inflammation but not during endotoxemia. The Journal of neuroscience : the official journal of the Society for Neuroscience 19:10923-10930.
Layé S (2010) Polyunsaturated fatty acids, neuroinflammation and well being. Prostaglandins, leukotrienes, and essential fatty acids 82:295-303.
Licinio J, Seibyl JP, Altemus M, Charney DS, Krystal JH (1993) Elevated CSF levels of interleukin-2 in neuroleptic-free schizophrenic patients. The American journal of psychiatry 150:1408-1410.
Masliah E, Heaton RK, Marcotte TD, Ellis RJ, Wiley CA, Mallory M, Achim CL, McCutchan JA, Nelson JA, Atkinson JH, Grant I (1997) Dendritic injury is a pathological substrate for human immunodeficiency virus-related cognitive disorders. HNRC Group. The HIV Neurobehavioral Research Center. Annals of neurology 42:963-972.
McCarthy M, Vidaurre I, Geffin R (2006) Maturing neurons are selectively sensitive to human immunodeficiency virus type 1 exposure in differentiating human neuroepithelial progenitor cell cultures. Journal of neurovirology 12:333-348.
Melis MP, Angioni E, Carta G, Murru E, Scanu P, Spada S, Banni S. Characterization of conjugated linoleic acid and its metabolites by RP-HPLC with diode array detector. Eur J Lipid Sci Technol 2001;103(9):617-21.
Melis M, Carta G, Pistis M, Banni S (2013a) Physiological role of peroxisome proliferator-activated receptors type alpha on dopamine systems. CNS & neurological disorders drug targets 12:70-77.
Melis M, Carta S, Fattore L, Tolu S, Yasar S, Goldberg SR, Fratta W, Maskos U, Pistis M (2010) Peroxisome proliferator-activated receptors-alpha modulate dopamine cell activity through nicotinic receptors. Biol Psychiatry 68:256-264.
Melis M, Scheggi S, Carta G, Madeddu C, Lecca S, Luchicchi A, Cadeddu F, Frau R, Fattore L, Fadda P, Ennas MG, Castelli MP, Fratta W, Schilstrom B, Banni S, De Montis MG, Pistis M (2013b) PPARalpha regulates cholinergic-driven activity of midbrain dopamine neurons via a novel mechanism involving alpha7 nicotinic acetylcholine receptors. J Neurosci 33:6203-6211.
Moolwaney AS, Igwe OJ (2005) Regulation of the cyclooxygenase-2 system by interleukin-1beta through mitogen-activated protein kinase signaling pathways: a comparative study of human neuroglioma and neuroblastoma cells. Brain research Molecular brain research 137:202-212.
Moya-Camarena SY, Vanden Heuvel JP, Blanchard SG, Leesnitzer LA, Belury MA (1999) Conjugated linoleic acid is a potent naturally occurring ligand and activator of PPARalpha. J Lipid Res 40:1426-1433.
Muller N, Schwarz MJ (2008) A psychoneuroimmunological perspective to Emil Kraepelins dichotomy: schizophrenia and major depression as inflammatory CNS disorders. European archives of psychiatry and clinical neuroscience 258 Suppl 2:97-106.
Okuda S, Saito H, Katsuki H (1994) Arachidonic acid: toxic and trophic effects on cultured hippocampal neurons. Neuroscience 63:691-699.
Pantelis C, Brewer W (1995) Neuropsychological and olfactory dysfunction in schizophrenia: relationship of frontal syndromes to syndromes of schizophrenia. Schizophrenia research 17:35-45.
Pariza MW, Park Y, Cook ME. The biologically active isomers of conjugated linoleic acid. Prog Lipid Res 2001;40(4):283-98.
Piscitelli F, Carta G, Bisogno T, Murru E, Cordeddu L, Berge K, Tandy S, Cohn JS, Griinari M, Banni S, et al. Effect of dietary krill oil supplementation on the endocannabinoidome of metabolically relevant tissues from high-fat-fed mice. Nutr Metab 2011;8:51.
Price G, Cercignani M, Chu EM, Barnes TR, Barker GJ, Joyce EM, Ron MA (2010) Brain pathology in first-episode psychosis: magnetization transfer imaging provides additional information to MRI measurements of volume loss. Neurolmage 49:185-192.
Puligheddu M, Pillolla G, Melis M, Lecca S, Marrosu F, De Montis MG, Scheggi S, Carta G, Murru E, Aroni S, Muntoni AL, Pistis M (2013) PPAR-Alpha Agonists as Novel Antiepileptic Drugs: Preclinical Findings. PLoS One 8:e64541.
Rakhshandehroo M, Knoch B, Muller M, Kersten S (2010) Peroxisome Proliferator-Activated Receptor Alpha Target Genes. PPAR research 2010:Article ID 612089.
Rao JS, Rapoport SI, Kim HW (2011a) Altered neuroinflammatory, arachidonic acid cascade and synaptic markers in postmortem Alzheimer's disease brain. Translational psychiatry 1:e31.
Rao JS, Ertley RN, Rapoport SI, Bazinet RP, Lee HJ (2007) Chronic NMDA administration to rats up-regulates frontal cortex cytosolic phospholipase A2 and its transcription factor, activator protein-2. Journal of neurochemistry 102:1918-1927.
Rao JS, Kellom M, Kim HW, Rapoport SI, Reese EA (2012) Neuroinflammation and synaptic loss. Neurochemical research 37:903-910.
Rao JS, Kim HW, Kellom M, Greenstein D, Chen M, Kraft AD, Harry GJ, Rapoport SI, Basselin M (2011b) Increased neuroinflammatory and arachidonic acid cascade markers, and reduced synaptic proteins, in brain of HIV-1 transgenic rats. Journal of neuroinflammation 8:101.
Rapoport SI, Ramadan E, Basselin M. Docosahexaenoic acid (DHA) incorporation into the brain from plasma, as an in vivo biomarker of brain DHA metabolism and neurotransmission. Prostaglandins Other Lipid Mediat 2011;96(1-4):109-13.
Rogers A, Schmuck G, Scholz G, Griffiths R, Meredith C, Schousboe A, Campiani G, Williams DC (2005) Improvements in an in-vitro assay for excitotoxicity by measurement of early gene (c-fos mRNA) levels. Archives of toxicology 79:129-139.
Rosenberger TA, Villacreses NE, Hovda JT, Bosetti F, Weerasinghe G, Wine RN, Harry GJ, Rapoport SI (2004) Rat brain arachidonic acid metabolism is increased by a 6-day intracerebral ventricular infusion of bacterial lipopolysaccharide. Journal of neurochemistry 88:1168-1178.
Rothwell NJ, Luheshi GN (2000) Interleukin 1 in the brain: biology, pathology and therapeutic target. Trends in neurosciences 23:618-625.
Someya S, Yamasoba T, Weindruch R, Prolla TA, Tanokura M (2007) Caloric restriction suppresses apoptotic cell death in the mammalian cochlea and leads to prevention of presbycusis. Neurobiol Aging 28:1613-1622.
Spijker J, de Graaf R, Bijl RV, Beekman AT, Ormel J, Nolen WA (2002) Duration of major depressive episodes in the general population: results from The Netherlands Mental Health Survey and Incidence Study (NEMESIS). The British journal of psychiatry : the journal of mental science 181:208-213.
Steiner J, Bielau H, Brisch R, Danos P, Ullrich O, Mawrin C, Bernstein HG, Bogerts B (2008) Immunological aspects in the neurobiology of suicide: elevated microglial density in schizophrenia and depression is associated with suicide. Journal of psychiatric research 42:151-157.
Terry-Lorenzo RT, Inoue M, Connor JH, Haystead TA, Armbruster BN, Gupta RP, Oliver CJ, Shenolikar S (2000) Neurofilament-L is a protein phosphatase-1-binding protein associated with neuronal plasma membrane and post-synaptic density. The Journal of biological chemistry 275:2439-2446.
Townsend LA, Malla AK, Norman RM (2001) Cognitive functioning in stabilized first-episode psychosis patients. Psychiatry research 104:119-131.
van Berckel BN, Bossong MG, Boellaard R, Kloet R, Schuitemaker A, Caspers E, Luurtsema G, Windhorst AD, Cahn W, Lammertsma AA, Kahn RS (2008) Microglia activation in recent-onset schizophrenia: a quantitative (R)-[11C]PK11195 positron emission tomography study. Biological psychiatry 64:820-822.
Venters HD, Dantzer R, Kelley KW (2000) A new concept in neurodegeneration: TNFalpha is a silencer of survival signals. Trends in neurosciences 23:175-180.
Wager-Smith K, Markou A (2011) Depression: a repair response to stress-induced neuronal microdamage that can grade into a chronic neuroinflammatory condition? Neuroscience and biobehavioral reviews 35:742-764.
Weinberger DR, Berman KF, Illowsky BP (1988) Physiological dysfunction of dorsolateral prefrontal cortex in schizophrenia. III. A new cohort and evidence for a monoaminergic mechanism. Archives of general psychiatry 45:609-615.
Williams JR, Leaver HA, Ironside JW, Miller EP, Whittle IR, Gregor A (1998) Apoptosis in human primary brain tumours: actions of arachidonic acid. Prostaglandins Leukot Essent Fatty Acids 58:193-200.
World Health Organization Dhwwimhmdde In.
Yagami T, Ueda K, Asakura K, Hata S, Kuroda T, Sakaeda T, Takasu N, Tanaka K, Gemba T, Hori Y (2002) Human group IIA secretory phospholipase A2 induces neuronal cell death via apoptosis. Molecular pharmacology 61:114-126.

## Claims

1. Ester of a phospholipid with conjugated linoleic acid or conjugated linoleic acid and docosahexaenoic acid wherein the conjugated linoleic acid is the c9,t11 isomer, for use in the treatment of neuroinflammatory and neurodegenerative disorders.

2. Ester according to claim 1, for use in the treatment according to claim 1, wherein said phospholipid is selected from the group consisting of phosphatidylcholine, phosphatidylethanolamine, phosphatidylserine.

3. Ester according to claim 1 or 2, for use in the treatment according to any one of claims 1-2, wherein the disorders are selected from the group consisting of psychotic disorders such as schizophrenia, schizophreniform disorder, schizoaffective disorder, or mood disorders such as major depression, dysthymic disorder.

4. Pharmaceutical composition comprising or consisting of an ester of a phospholipid with conjugated linoleic acid or conjugated linoleic acid and docosahexaenoic acid as active ingredient wherein the conjugated linoleic acid is the c9,t11 isomer, in association with one or more pharmaceutically acceptable excipients and/or adjuvants, for use in the treatment of neuroinflammatory and neurodegenerative disorders .

5. Pharmaceutical composition according to claim 4 for the use according to claim 4, wherein said phospholipid is selected from the group consisting of phosphatidylcholine, phosphatidylethanolamine, phosphatidylserine.

6. Pharmaceutical composition according to claim 4 or 5, for the use according to claim 4, further comprising antipsychotic and/or antidepressant drugs.

7. Pharmaceutical composition according to claims 4-6, for the use according to claim 4, wherein said pharmaceutical composition is a therapeutic composition or a food supplement.

8. Pharmaceutical composition according to claims 4-7, for the use according to claim 4, wherein the disorders are selected from the group consisting of psychotic disorders such as schizophrenia, schizophreniform disorder, schizoaffective disorder, or mood disorders such as major depression, dysthymic disorder.

## Patentansprüche

1. Ester eines Phospholipids mit konjugierter Linolsäure oder konjugierter Linolsäure und Docosahexaensäure, während die konjugierte Linolsäure das Isomer c9, t11 zur Verwendung bei der Behandlung von neuroinflammatorischen und neurodegenerativen Erkrankungen ist.

2. Ester nach Anspruch 1 zur Verwendung bei der Behandlung nach Anspruch 1, wobei das gesagte Phospholipid ausgewählt ist aus der Gruppe, die durch Phosphatidylcholin, Phosphatidylethanolamin, Phosphatidylserin gebildet wird.

3. Ester nach Anspruch 1 oder 2 zur Verwendung bei der Behandlung nach einem der Ansprüche 1 bis 2, wobei die Störungen aus der Gruppe ausgewählt sind, die durch psychotische Störungen wie Schizophrenie, schizophreniforme Störung, schizoaffektive Störung oder Stimmungsstörungen wie schwere Depression, dysthymische Störung, gebildet wird.

4. Pharmazeutische Zusammensetzung umfassend oder gebildet aus einem Ester eines Phospholipids mit konjugierter Linolsäure oder konjugierter Linolsäure und Docosahexaensäure als Wirkstoff, wobei die konjugierte Linolsäure ist das Isomer c9, t11 in Verbindung mit einem oder mehreren pharmazeutisch verträglichen Hilfsstoffen und/oder Adjuvantien, zur Verwendung bei der Behandlung von neurinflammatorischen und neurodegenerativen Störungen.

5. Pharmazeutische Zusammensetzung nach Anspruch 4 zur Verwendung nach Anspruch 4, wobei das gesagte Phospholipid ausgewählt ist aus der Gruppe, die durch Phosphatidylcholin, Phosphatidylethanolamin und Phosphatidylserin gebildet wird.

6. Pharmazeutische Zusammensetzung nach Anspruch 4 oder 5 zur Verwendung nach Anspruch 4, ferner umfassend Antipsychotika und/oder Antidepressiva.

7. Pharmazeutische Zusammensetzung nach Anspruch 4-6 zur Verwendung nach Anspruch 4, wobei die gesagte pharmazeutische Zusammensetzung eine therapeutische Zusammensetzung oder ein Nahrungsergänzungsmittel ist.

8. Pharmazeutische Zusammensetzung nach Anspruch 4-7 zur Verwendung nach Anspruch 4, wobei die Störungen ausgewählt sind aus der Gruppe bestehend aus psychotischen Störungen wie Schizophrenie, schizophreniforme Störung, schizoaffektive Störung oder humoralen Störungen wie schwerer Depression, dysthymischer Störung.

## Revendications

1. Ester d'un phospholipide avec de l'acide linoléique conjugué ou de l'acide linoléique conjugué et de l'acide docosahexaénoïque, tandis que l'acide linoléique conjugué est l'isomère c9, t11, destiné à être utilisé dans le traitement des troubles neuroinflammatoires et neurodégénératifs.

2. Ester selon la revendication 1, pour l'utilisation du traitement selon la revendication 1, dans lequel ledit phospholipide est choisi dans le groupe formé par la phosphatidylcholine, la phosphatidyléthanolamine, la phosphatidylsérine.

3. Ester selon la revendication 1 ou 2, pour l'utilisation du traitement selon l'une des revendications 1 à 2, dans lequel les troubles sont choisis dans le groupe formé par les troubles psychotiques tels que la schizophrénie, les troubles schizophréniformes, les troubles schizo-affectifs ou les troubles de l'humeur, tels que une dépression sévère, un trouble dysthymique.

4. Composition pharmaceutique comprenant ou formée par un ester d'un phospholipide avec de l'acide linoléique conjugué ou de l'acide linoléique conjugué et d'un acide docosahexaénoïque comme ingrédient actif, dans laquelle l'acide linoléique conjugué est l'isomère c9, t11 en association avec un ou plusieurs excipients et/ou adjuvants pharmaceutiquement acceptables, destinés à être utilisés dans le traitement de troubles neurinflammatoires et neurodégénératifs.

5. Composition pharmaceutique selon la revendication 4 pour l'utilisation selon la revendication 4, dans laquelle ledit phospholipide est choisi dans le groupe formé par la phosphatidylcholine, la phosphatidyléthanolamine et la phosphatidylsérine.

6. Composition pharmaceutique selon la revendication 4 ou 5 pour l'utilisation selon la revendication 4, comprenant en outre des médicaments antipsychotiques et/ou antidépresseurs.

7. Composition pharmaceutique selon les revendications 4 à 6 pour l'utilisation selon la revendication 4, dans laquelle ladite composition pharmaceutique est une composition thérapeutique ou un complément alimentaire.

8. Composition pharmaceutique selon la revendication 4 à 7 pour l'utilisation selon la revendication 4, dans laquelle les troubles sont choisis dans le groupe formé par les troubles psychotiques tels que la schizophrénie, les troubles schizophréniformes, les troubles schizo-affectifs ou les troubles de l'humeur, tels que une dépression sévère, un trouble dysthymique.
